# EUROPEAN PATENT SPECIFICATION

(11) **EP 3 695 814 B1**
(45) Date of publication and mention of the grant of the patent: **22.07.2026**
(21) Application number: 19738408.4
(22) Date of filing: 11.01.2019
(51) Int. Cl.: A61F 2/95, A61F 2/966

(54) **HANDLE FOR MEDICAL IMPLANT DELIVERY SYSTEM**
GRIFF FÜR EIN MEDIZINISCHES IMPLANTATEINFÜHRUNGSSYSTEM
POIGNÉE POUR SYSTÈME DE POSE D'IMPLANT MÉDICAL

(30) Priority: 12.01.2018 CN 201810030189
(43) Date of publication of application: 19.08.2020
(73) Proprietor: Shanghai Microport Endovascular Medtech Co., Ltd., Shanghai 201318 (CN)
(72) Inventor: PENG, Dadong, Shanghai 201318 (CN); JIAO, Wenhui, Shanghai 201318 (CN); ZHU, Qing, Shanghai 201318 (CN); TU, Chunlin, Shanghai 201318 (CN); YUAN, Zhenyu, Shanghai 201318 (CN)
(74) Representative: Loo, Chi Ching
(86) International application number: PCT/CN2019/071291
(87) International publication number: WO 2019/137456

(56) References cited:
- EP-A1- 3 395 301
- WO-A1-2017/107990
- CN-A- 101 151 003
- CN-A- 103 079 500
- CN-A- 105 530 893
- CN-Y- 2 902 201
- CN-Y- 201 194 835
- US-A1- 2014 046 428
- US-A1- 2016 158 050

## Description

### Field of the Invention

The present invention relates to a handle of a delivery system, in particular relates to a handle for a medical implant delivery system, such as laminated stent, which is applied to the field of endovascular treatment.

### Background of the Invention and Discussion of the Prior Art

Thoracic aortic aneurysm or dissection is a fatal vascular surgical disease, and its incidence is not low. Before the 1990s, traditional surgical treatment methods have been used in medicine, which is difficult, invasive, and has many complications. Until 1994, Dake et al. used endovascular laminated stent for Stanford B-type aortic dissection for the first time, and the interventional surgery was greatly developed.

The laminated stent is released in the segment of the vessel to be repaired, and a delivery system is required. The operation of the delivery system generally includes several steps such as introduction, release, and withdrawal. The accurate and effective operation of these steps will greatly improve the safety and positioning performance of the laminated stent. Medtronic has applied a patent document named "a catheter handle for the delivery system of prosthesis" with application number CN201180023910, which refers to the rotary release mechanism of the handle. US 2014/046428A1 and WO 2017/107990 A1 disclose stent delivery systems and associated methods. However, the technical structure of the handle assembly is too simple, and the function cannot effectively prevent misoperation. The main disadvantages in the prior arts are as follows:
(1) The operation of the delivery system generally includes several steps such as introduction, release, and withdrawal. The accurate and effective operation of the above steps will greatly improve the safety and positioning performance of the laminated stent.

In the prior arts it often appears all kinds of misoparation in the release state in the delivery system.

Case 1: In the process of rotating the handle to withdraw the outer tube, the operation was reversed by mistake, the outer tube was retrograde, and the released laminated section was jacked up by the outer sheath tube, resulting in the displacement of the stent.

Case 2: The bare section of the stent will be rear released before the laminated section released completely, and then the outer tube will be withdrawn to release the remaining laminated section, during the release of the last few laminated section, it is easy to drive the stent and lead it to the displacement.

Case 3: If the delivering system includes steps to pre-release the laminated stent to the casing pipe before releasing the laminated stent, there is also a risk of case 1 and case 2.

Case 4: There is also a risk that the stent will be pulled and displaced in the withdrawing step due to forgetting to rear release and then withdrawing the inner tube.

(2) Most of the existing release systems are two-step release, that is, the stent is positioned in the outer sheath and then released the outer sheath, and then rear released. Because the outer sheath is hard, the stent will become soft during the release process, and the blood vessels will gradually return to shape as the delivery system becomes soft, and the stent will have relative displacement, resulting in inaccurate positioning. In addition, the operation gives the operator the less opportunity to adjust, and the higher experience of the operator is required.

(3) The low profile design of the outer sheath will increase the release resistance compared with the previous design, which is easy to drive the stent during release, leading the stent to displacement.

The external diameter of the imported or domestic large artery laminated stent system sold on the market is mostly 22F~24F, while the femoral artery approach in Asians is generally narrow. There are many cases where the stent system cannot reach the lesion smoothly due to the above reasons, so they give up the endovascular surgery program. Even if they barely reach the lesion, they will also be caused by the injury of the femoral artery, iliac artery and other access vessels by the instrument source series of complications. In order to adapt to more patients with narrow access and effectively resist the complications caused by the above reasons, the new technology compresses the stent in the outer sheath of 20F, which leads to the increase of release resistance. When releasing, it is easy to drive the stent and lead to stent displacement.

It can be seen from the above that most of the above possible adverse phenomena are caused by the design of the delivering system, which leaves more room for the operator to play freely. Therefore, it is necessary to provide a fool-proofing catheter handle for the release system of the laminated stent, which can strictly fix the operation sequence of each step of the delivering system. If the previous step is not completed, the next step cannot be started, so as to achieve to reduce misoperation.

### Summary of the Invention

The technical problem to be solved by the present invention is to provide a handle for a medical implant delivery system, which can effectively prevent the error of the release step, thereby avoiding the misoperation of the operator, and greatly improving the safety of product use.

The technical solution adopted by the present invention to solve the above technical problems is to provide a handle for a medical implant delivery system, as claimed in claim 1

Preferably, a control panel is rotatably provided on the outer circumferential surface of the handle rear end, a laminated pull ring and a rear release pull ring are sequentially provided on the handle rear end along the rotation direction of the control panel, an inner tube end connector is internally provided in the handle rear end, the laminated pull ring and the rear release pull ring are detachably connected with the control panel.

Preferably, the control panel has a hollow cylindrical shape, a second boss is provided inside the control panel, a second groove matching with the second boss is provided on the laminated pull ring and the rear release pull ring, a unique notch through which the proximal end laminated pull ring and the rear release pull ring can pass is formed on the second boss inside the control plate, an one-way inverted tooth is provided inside the control plate.

Preferably, a baffle plate protruding inward is provided at the proximal or distal end at the notch of the control panel, when the baffle plate rotates to the laminated pull ring or the rear release pull ring, the baffle plate is against the laminated pull ring or the rear release pull ring making the control panel stop rotating, the notch of the second boss of the control panel is aligned with the laminated pull ring or the rear release pull ring.

Preferably, a rotatable retract control panel is provided between the control panel and the handle rear end, the rotatable retract control panel is detachably connected to the handle rear end by a buckle.

Preferably, a groove is provided on the buckle, the retract control panel snaps into the groove on the buckle, the retract control panel is ring-shaped, a second notch and a third notch for the distal end of the buckle to pass through are provided inside the retract control panel, the shape of the second notch matches the proximal end of the laminated pull ring or the rear release pull ring, when the laminated pull ring or the rear release pull ring is inserted into the second notch, the position of the third notch and the buckle are staggered..

Preferably, when the retract control panel rotates to the third notch and overlaps with the buckle, the retract control panel is separated from the buckle making the inner tube end connector is in a retractable state.

Preferably, the surface of the outer tube is coated with a hydrophilic coating.

Compared with the prior art, the beneficial effects of the present invention are as follows: the handle for a medical implant delivery system provided by the present invention, the thread rotation release is used to skillfully convert the linear tension into rotary torque, which makes the feel of the release more comfortable and the release performance safer and more stable. By setting the anti-reverse rotation switch and the anti-reverse rotation elastic plate, the release step can be effectively prevented from errors, thereby avoiding the misoperation of the operator, and greatly improving the safety of product use. Moreover, the stepwise positioning and release give the operator more opportunities for adjustment, the positioning of the stent is more accurate, and the stent is not easily displaced when released.

### Brief Description of the Drawings

Fig.1a is a schematic view illustrating the structure of a handle for a medical implant delivery system according to one embodiment of this invention.
Fig. 1b is a partially enlarged schematic view illustrating the structure of the tail end of the handle in Figure 1a.
Fig.2 is a schematic view illustrating the structure of the connection of the front handle, the rear handle and the handle rear end according to one embodiment of this invention.
Fig.3 is a schematic view illustrating the anti-reverse structure of the connection between the front handle and the rear handle according to one embodiment of this invention.
Fig.4 is a cross-sectional view in the axial direction from the vicinity of the control panel to the rear end region of the handle, which is a schematic diagram showing the side connection structure of the control panel and the pull ring of this invention.
Fig.5 is a schematic view illustrating the structure of the control panel of this invention.
Fig.6 is a schematic view illustrating the structure of the laminated pull ring of this invention.
Fig.7 is a schematic view illustrating the front connection structure of the control panel and the pull ring of this invention.
Fig.8 is a cross-sectional view taken along line A-A in Fig.7.
Fig.9 is a schematic view illustrating the structure after the control panel rotates through the laminated pull ring, and the baffle plate is turned to release the boss of the pull ring to stop of this invention.
Fig.10 is a schematic view illustrating the structure of the retract control panel of this invention.
Fig.11 is a schematic view illustrating the connection between the retract control panel and the pull ring (sectional view perpendicular to the axis).
Fig.12 is a schematic view illustrating of the side connection of the retract control panel and the pull ring of this invention.
Fig.13a and Fig.13b are schematic view illustrating the inclined delivering system of this invention.
Fig.14a and Fig.14b are schematic view illustrating injecting heparin saline into the inner tube from the inner tube end connector and flowing out from the tapered head of this invention.
Fig.15 is a schematic view illustrating introducing the delivery system into the patient along the super-hard guide wire of this invention.
Fig.16 is a schematic view illustrating fixing the front handle after turning on the anti-reverse rotation switch and rotating the rear handle clockwise.
Fig.17 is a schematic view diagram of adjusting the development point of the proximal end of the laminated stent using the handle of the laminated stent release system of this invention.
Fig.18a, Fig.18b, and Fig.18c are schematic illustrating rotating and releasing the laminated section of the laminated stent using the handle of this invention.
Fig.19a, Fig.19b and Fig.19c are schematic illustrating rotating and releasing the naked section of the laminated stent using the handle of this invention.
Fig.20a, Fig.20b and Fig.20c are schematic illustrating fully merging the distal end of the tapered head with the developing ring and withdrawing the delivery system from the body using the handle of this invention.

In the pictures:
1-a guide rod
2-a sliding block
3-an anti-reverse rotation elastic plate
4-a tapered head
5-a developing ring
6-an outer tube
7-a stress diffusion tube
8-a front handle
9-a check valve
10-an infusion tube
11-a control panel
12-a laminated pull ring
13-an inner tube end connector
14-a rear release pull ring
15-a handle rear end
16-a rear handle
17-an anti-reverse rotation switch
18-a retract control panel
19-an end cap
20-an inner tube locking cap
21-a baffle plate cap
22-a baffle plate
23-a first boss
24-a first groove
25-a second boss
26-a first notch
27-a through-hole
28-a third boss
29-a second notch
30-a third notch
31-a buckle
32-a second groove
33-a one-way inverted tooth

### Detailed Description of the Invention

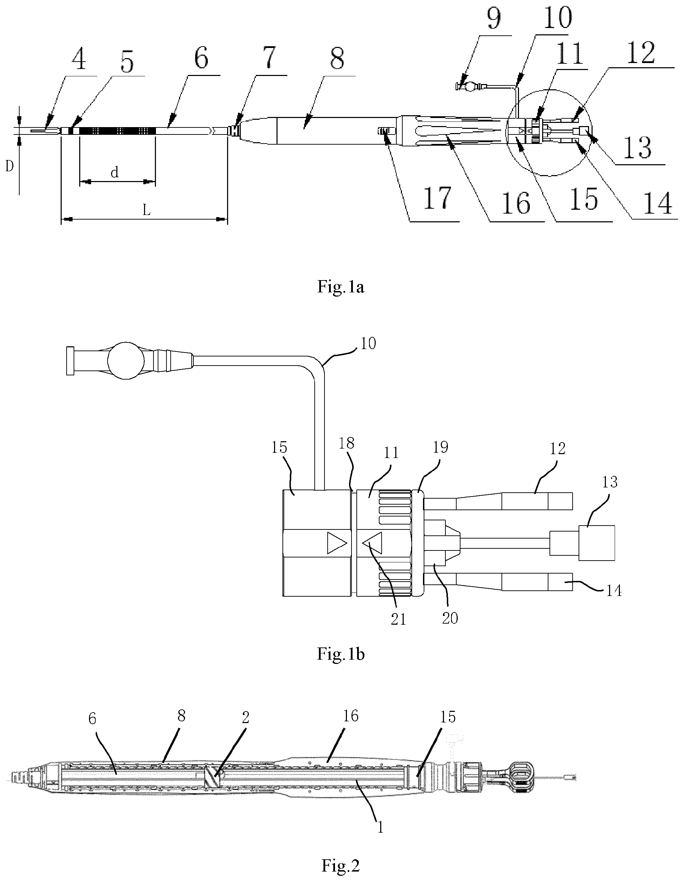

It should be noted: in the present invention, the proximal end refers to the end near the patient, sometimes also called the front end, the distal end refers to the end away from the patient, sometimes also called the back end. Take Fig.2 as an example, the proximal end (front end) is the left end of Fig.2, the distal end (back end) is the right end of Fig.2.

The invention will now be further described below with reference to the accompanying drawings and examples.

Fig.1a is a schematic view illustrating the structure of a handle for a medical implant delivery system according to one embodiment of this invention. Fig.1b is a partially enlarged schematic view illustrating the structure of the tail end of the handle in Figure 1a.

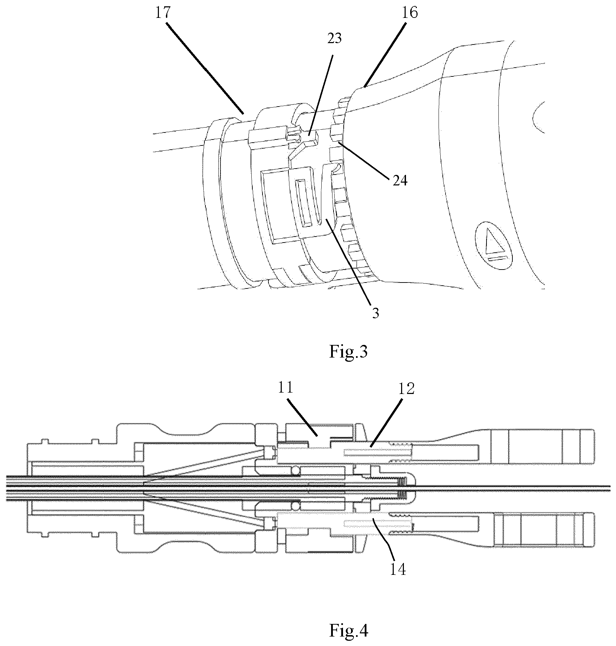

As shown in Fig.1a and Fig.1b, the handle for a medical implant delivery system provided by this invention, comprising a front handle 8 and a rear handle 16, the front handle 8 and the rear handle 16 are internally provided with a guide rod 1, the front handle 8 and a handle rear end 15 are fixedly connected as a whole by means of the guide rod 1, an outer tube 6 and the sliding block 2 and are fixedly connected as a whole by means of an outer tube connecting part. The effective length of the outer tube 6 is L, the diameter of the outer tube is D, and the length of the stent is d, a stress diffusion tube 7 can be provided between the outer tube 6 and the front handle 8. The sliding block 2 has external thread, the rear handle 16 can use an engagement slot of the front handle 8 and the handle rear end 15 as a pivot point to rotate, the rear handle 16 has an internal thread matching the external thread of the sliding block 2, when the rear handle 16 rotates clockwise, the sliding block 2 will drive the outer tube 6 to slide distally on the guide rod 1, so as to release the medical implants, such as a laminated stent. As shown in Fig.3, an anti-reverse rotation switch 17 and an anti-reverse rotation elastic plate 3 that limits a rotating direction of the rear handle 16 are provided at the point of connection of the front handle 8 and the rear handle 16.

As shown in Fig.3, when the anti-reverse rotation switch 17 of this invention is closed, the first boss 23 of the anti-reverse rotation switch 17 is inserted into the first groove 24 of the rear handle 16, and then the rear handle 16 cannot be rotated, ensuring that the stent will not be erroneously operated during transportation and use. After the anti-reverse rotation switch 17 is turned on, the first boss 23 of the anti-reverse rotation switch 17 and the first groove 24 of the rear handle 16 are separated, and then the rear handle 16 can be rotated, but under the restriction of the anti-reverse rotation elastic plate 3, the rear handle 16 can only rotated clockwise, not counterclockwise. In this embodiment, the anti-reverse rotation elastic plate has an inclined arm with elasticity that can swing around the pivot point in a small range. There is a small protrusion at the end of the inclined arm which is inclined on one side and straight on the other side. When the rear handle 16 rotates clockwise, the inclined arm will be pressed by the teeth formed by the first groove 24 at the proximal end of the rear handle 16 and swing toward the proximal end, so that the rear handle 16 can be rotated smoothly. When the rear handle 16 rotates counterclockwise, it cannot be rotated due to the blocking of the small protrusion at the end of the inclined arm and the supporting force of the inclined arm. In this embodiment, there are a plurality of first grooves 24 arranged in a circle around the circumference of the proximal end of the rear handle 16.

Rotate the rear handle 16 clockwise until the developing ring 5 in the outer tube is far away from the distal end of the laminated stent. If the development point is greater than 10 mm or the handle is turned to the limit, the anti-reverse rotation switch 17 can be turned to the distal end, at this time, the rear handle 16 cannot be rotated.

As shown in Fig.1b and Fig.4, the outer circumferential surface of the handle end 15 of this invention is provided with a retract control panel 18 and a control panel 11. The handle rear end 15 is provided with a laminated pull ring 12 and rear release pull ring 14 in turn along the direction of introduction and rotation of the control panel 11, the handle rear end 15 of the handle is provided with an inner tube end connector 13. The inner tube end connector 13 is fixed by an end cap 19 and an inner tube locking cap 20.

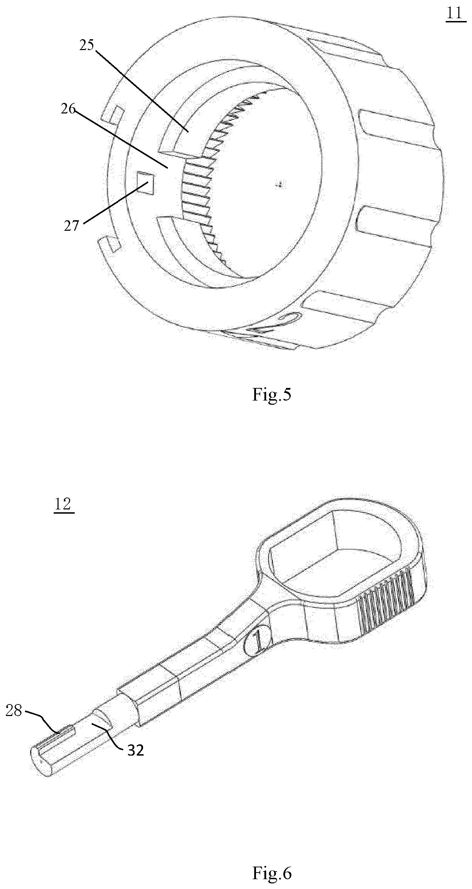

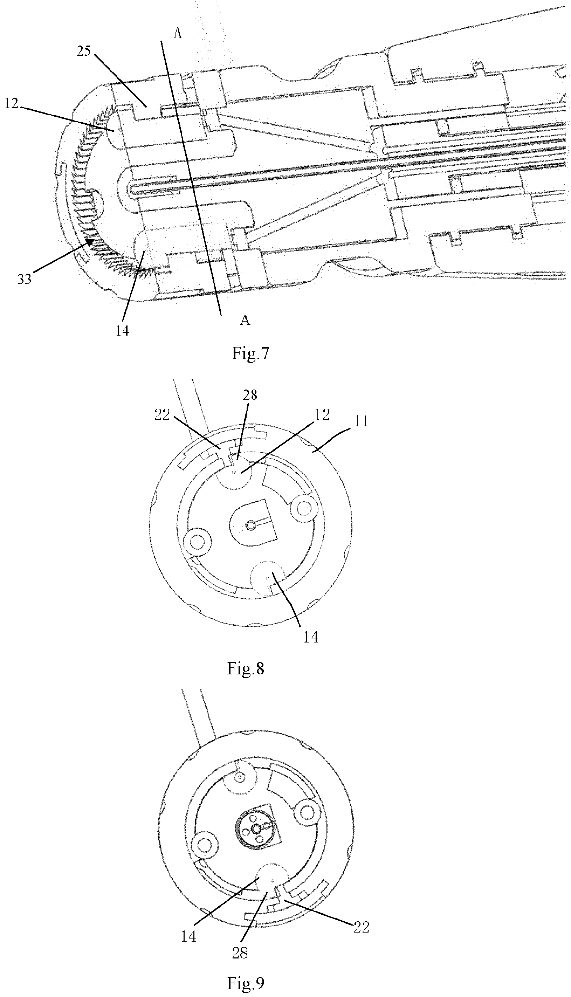

As shown in Fig.5, a second boss 25 is provided inside the control panel 11, a unique notch is provided on the second boss 25. A first notch 26 is as shown in Fig.5, there is a through-hole 27 in the center of the front end of the first notch 26, a baffle plate 22 protrudes from the through-hole 27. As shown in Fig.6, a second groove 32 matching with the second boss 25 is provided on the middle of the laminated pull ring 12, a third boss 28 is provided on the front end of the pull ring, the height of the third boss 28 is less than or equal to the depth of the first notch 26. The shape of the rear release pull ring 14 is the same as the laminated pull ring 12. As shown in Fig.7, in the initial state (when the release is not started), the second boss 25 of the control panel 11 is inserted into the second groove 32 in the middle of the pull ring. Due to the limitation of the third boss 28 at the front end of the pull ring, the pull ring cannot be pulled. When starting the release operation, turn the control panel 11 clockwise, only when the control panel 11 rotates to a specific position, that is, when the first notch 26 is aligned with the third boss 28 of the pull ring, the pull ring can be pulled back. The distal end of the control panel 11 has a one-way inverted tooth 33, which can only rotate clockwise.

Refer to Fig.8 for the determination of the above specific position, Fig.8 is a cross-sectional view taken along line A-A (at the front end of the second boss) in Fig.7. A baffle plate 22 is provided in the middle position of the notch of the control panel 11, when the control panel 11 rotates to the laminated pull ring 12, The baffle plate 22 hits the third boss 28 of the laminated pull ring 12 to the limit and must stop. At this time, the notch on the second boss 25 of the control panel 11 is exactly aligned with the third boss 28 of the laminated pull ring 12 which can be pulled out smoothly. As shown in Fig.9, when the baffle plate 22 rotates to the third boss 28 of the rear release pull ring 14 to the limit, it must be stopped. At this time, the notch on the second boss 25 of the control panel 11 is aligned exactly with the third boss 28 of the rear release pull ring 14 which can be pulled out and released. In this way, it can fix the operation steps in the operation process and prevent misoperation.

In other embodiments, the third boss 28 on the pull ring may have other shapes, it is also possible that there is no third boss 28 and the front end of the pull ring is a complete cylinder, as long as the position of the first notch 26 is directly opposite to the pull ring when it abuts the baffle plate 22. The baffle plate 22 can be provided at the distal end of the position of the first notch 26 or both at the distal end and proximal end, as long as it can be countered with the pull ring when turning to a specific position.

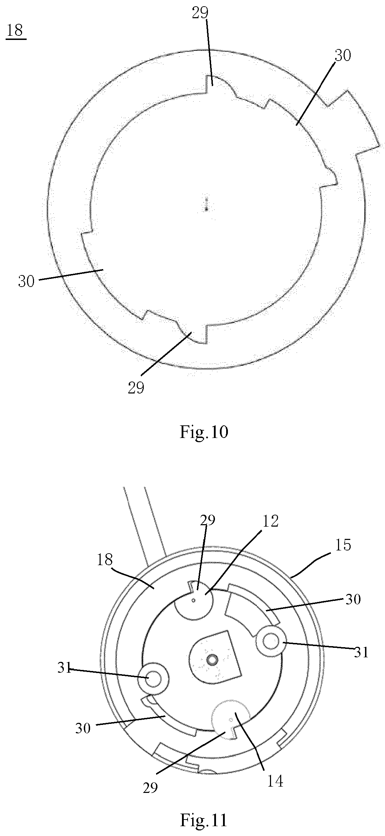

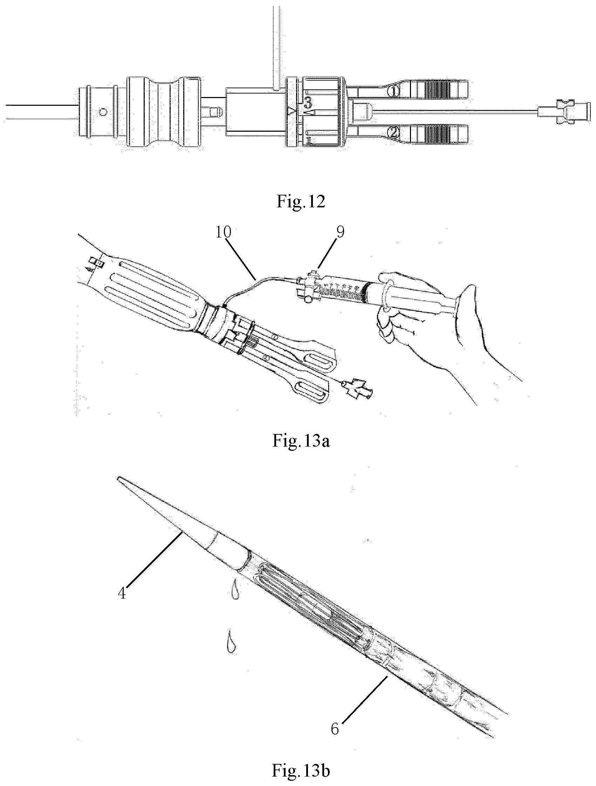

As shown in Fig.10 and Fig.11, there are 2 groups of notches in the retract control panel 18: the second notch 29 and the third notch 30, each group has two relative notches. As shown in Fig.11 and Fig.12, since the laminated pull ring 12 and the third boss 28 of the rear release pull ring 14 are inserted into the second notch 29 on the retract control panel 18, the retract control panel 18 cannot be rotated if the above two pull rings are not pulled out. When laminated pull ring 12 and the rear release pull ring 14 are all pulled out, the retract control panel 18 can be pushed to rotate by the proximal end of the baffle plate cap 21 when continue to rotate the control panel 11, due to the loss of the fixation of the laminated pull ring 12. A buckle 31 is provided at the end of the handle, a groove is provided in the middle of the buckle 31, the retract control panel 18 is stuck in the slot of the buckle 31, and when the pull ring is inserted into the second notch 29, the positions of the third notch 30 and the buckle 31 do not overlap, so that the buckle 31 cannot be pulled out to the front end. After the laminated pull ring 12 and the rear release pull ring 14 are pulled out, with the rotation of the retract control panel 18, when the third notch 30 on the retraction control disk 18 is turned to the buckle 31, the retraction control disk 18 is detached from the end of the handle 15, and the inner tube end connector 13 is pulled to retract the inner tube.

The second notch 29 on the retract control panel 18 can be other shapes, as long as it can match the proximal end of the pull ring. The number of the buckle may be one or more, as long as it matches the number of the third notch 30.

In summary, the handle of the medical implant delivery system provided by the present invention can fix the operation sequence of each step of the delivery system. If the previous step is not completed, the latter step cannot be started, thereby achieving the purpose of reducing misoperation. Step by step positioning and release give the operator more opportunities to adjust, the positioning of the stent is more accurate, and the stent is not easy to move when released.

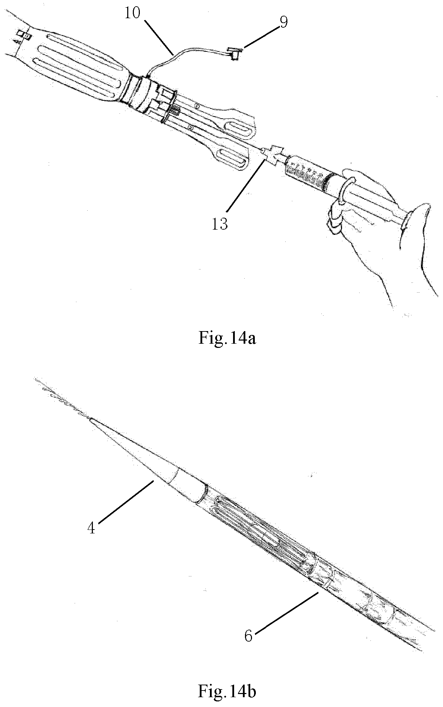

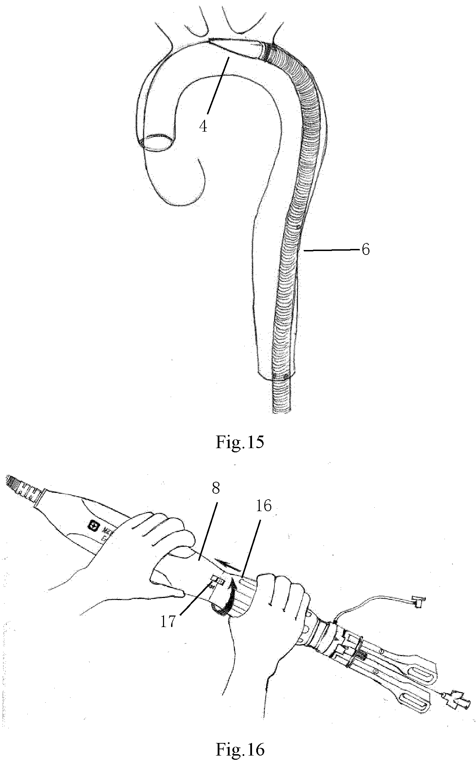

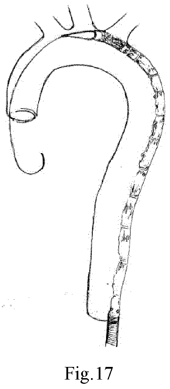

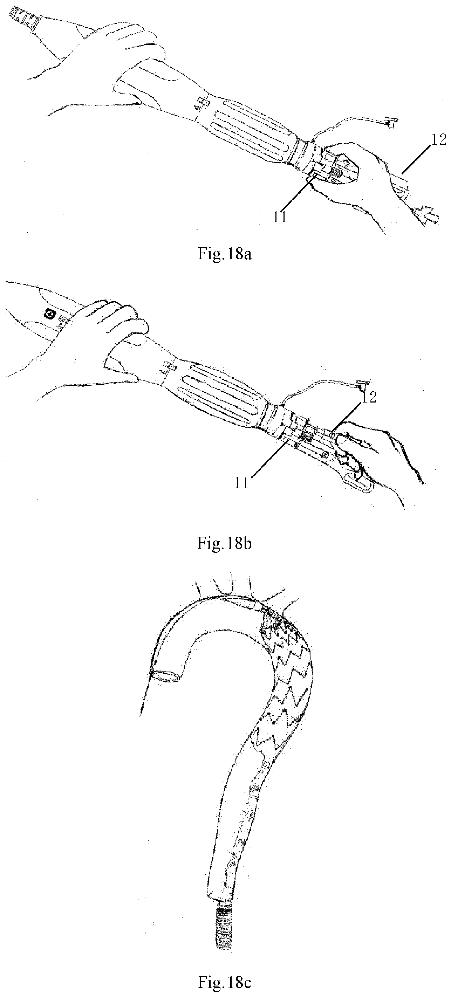

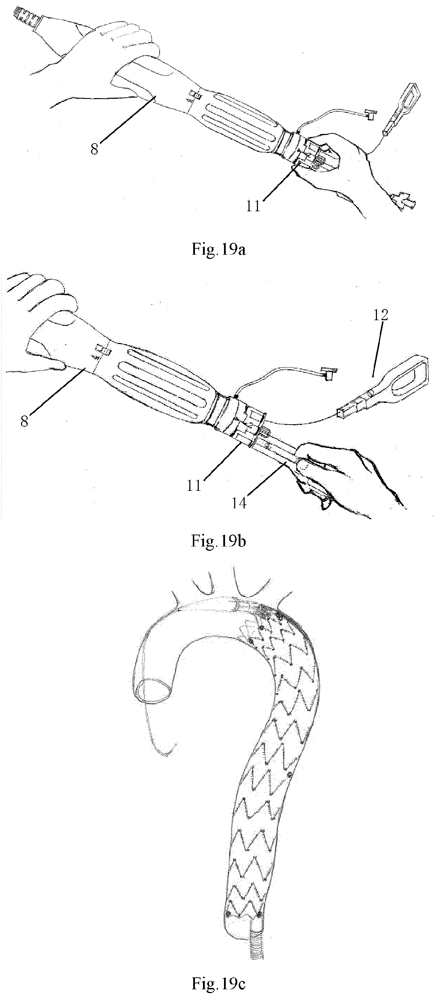

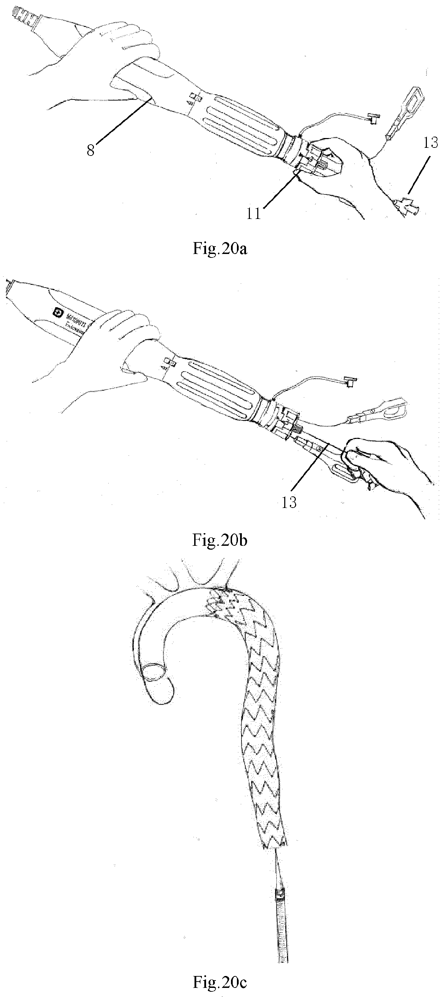

The specific use process, not in accordance with the present invention, of the handle of the medical implant release system of the present invention is given below.
1. As shown in Fig.13a and Fig.13b, inclining the delivery system, tilting the delivering system so that the tapered head 4 is inclined upward, and injecting heparin salt water into the delivering system from the check valve 9. At the same time, flicking the outer tube 6 of the delivering system to make the heparin salt water flow out of the tapered head 4, and closing the check valve 9. As shown in Fig.14a and Fig.14b, the check valve 9 is provided in an infusion tube 10, and injecting heparin salt water into the inner tube from the inner tube end connector to make the heparin salt water flow out of the tapered head 4.
2. As shown in Fig. 15, 0.035 "ultra-hard guide wire is introduced to the ascending aorta. The ultra-hard guide wire is pushed into the body of the patient along the ultra-hard guide wire. Normal saline is used to infiltrate the surface of the tapered head 4 and outer tube 6 to activate the hydrophilic coating before introduction of blood vessels. With the help of the X-ray imaging equipment and the development points on the laminated stent, the system can reach the predetermined position.
3. As shown in Fig. 16, push the anti-reverse rotation switch 17 towards the proximal end according to the arrow direction of the front handle 8. Fix the front handle 8, rotate the rear handle 16 clockwise according to the arrow direction of the rear handle, and monitor the movement of the developing ring 5 in the outer tube 6 with the help of the X-ray display screen until the developing ring 5 in the outer tube is more than 10 mm away from the distal end of the laminated stent or the handle is turned to the limit. Close the anti-reverse rotation switch 17 to the distal end in the reverse arrow direction of the front handle.
4. As shown in Fig.17, adjust the front and back positions of the development points at the proximal end of the laminated stent to ensure that the development points coincide with the target positions. Four development points can be provided in the circumferential direction of the proximal end of the laminated stent, please position the laminated stent according to the development points closest to the target.
5. As shown in Fig.18a, Fig.18b and Fig.18c, fix the front handle 8, rotate the control panel 11 clockwise, align the arrow "1" with the arrow at the end of the handle (the limit position), and pull the yellow laminated pull ring 12 until the laminated section of the laminated stent completely pops out.
6. As shown in Fig. 19a, Fig.19b and Fig.19c, fix the front handle 8, rotate the control panel 11 clockwise, align the arrow "2" with the arrow at the end of the handle (the limit position), and pull the green rear release pull ring 14 until the naked section/proximal end pops out.
7. As shown in Fig.20a, Fig.20b and Fig.20c, fix the front handle 8, rotate the control panel 11 clockwise, align the arrow "3" with the narrow at the end of the handle (the limit position), and pull the inner tube end connector 13 until the distal end of the tapered head 4 completely coincided with the developing point 5, then withdraw the delivery system from the body.

The effective fool-proofing handle provided by the present invention adopts a step-by-step positioning and releasing system. The laminated stent compressed in the outer tube 6 of the composite material with the coil wire can easily pass through the bending lesion. The initial positioning is completed according to the development point on the stent, and it is easily released in the aortic arch or lesion. After the relatively hard outer sheath is released, the stent is still flexibly bound by the guide wire and coil. Then, the laminated stent is accurately positioned with the aid of the development point of the laminated stent, the laminated segment of the stent is released, then the bare segment of the stent is released, and finally the delivery system is withdrawn by the tapered head 4 from the body.

The invention shall be protected by the scope of the claims.

## Claims

1. A handle for a medical implant delivery system, comprising a front handle (8) and a rear handle (16), the front handle (8) and the rear handle (16) are internally provided with a guide rod (1), a sliding block (2) is provided on the guide rod (1), the front handle (8) and a handle rear end (15) are fixedly connected as a whole by means of the guide rod (1), the sliding block (2) and an outer tube (6) are fixedly connected as a whole by means of an outer tube connecting part, the sliding block (2) and the rear handle (16) are rotatably connected, the rear handle (16) rotates to enable the sliding block (2) to drive the outer tube (6) to slide toward distal end on the guide rod (1), wherein an anti-reverse rotation switch (17) and an anti-reverse rotation elastic plate (3) that limits a rotating direction of the rear handle (16) are provided at the point of connection of the front handle (8) and the rear handle (16),
a first boss (23) is provided on the anti-reverse rotation switch (17), a first groove (24) matching with the first boss (23) is provided on the rear handle (16), when the anti-reverse rotation switch (17) is closed, the first boss (23) of the anti-reverse rotation switch (17) is inserted into the first groove (24) of the rear handle (16), and then the rear handle (16) cannot be rotated, after the anti-reverse rotation switch (17) is turned on, the first boss (23) of the anti-reverse rotation switch (17) and the first groove (24) of the rear handle (16) are separated, and then the rear handle (16) can be rotated,
the anti-reverse rotation elastic plate (3) has an inclined arm with elasticity , there is a protrusion at the end of the inclined arm which is inclined on one side and straight on the other side, the protrusion is inserted into the first groove (24) at the proximal end of the rear handle (16), wherein there are a plurality of the first grooves (24) arranged in a circle around a circumference of the proximal end of the rear handle (16).

2. The handle for a medical implant delivery system of claim 1, wherein a control panel (11) is rotatably provided on the outer circumferential surface of the handle rear end (15), a laminated pull ring (12) and a rear release pull ring (14) are sequentially provided on the handle rear end (15) along the rotation direction of the control panel (11), an inner tube end connector (13) is internally provided in the handle rear end (15), the laminated pull ring (12) and the rear release pull ring (14) are detachably connected with the control panel (11).

3. The handle for a medical implant delivery system of claim 2, wherein the control panel (11) has a hollow cylindrical shape, a second boss (25) is provided inside the control panel (11), a second groove (32) matching with the second boss (25) is provided on the laminated pull ring (12) and the rear release pull ring (14), a unique notch through which the proximal end laminated pull ring (12) and the rear release pull ring (14) can pass is formed on the second boss (25) inside the control plate (11), an one-way inverted tooth (33) is provided inside the control plate (11).

4. The handle for a medical implant delivery system of claim 3, wherein a baffle plate (22) protruding inward is provided at the proximal or distal end at the notch of the control panel (11), when the baffle plate (22) rotates to the laminated pull ring (12) or the rear release pull ring (14), the baffle plate (22) is against the laminated pull ring (12) or the rear release pull ring (14) making the control panel (11) stop rotating, the notch of the second boss (25) of the control panel (11) is aligned with the laminated pull ring (12) or the rear release pull ring (14).

5. The handle for a medical implant delivery system of claim 4, wherein a rotatable retract control panel (18) is provided between the control panel (11) and the handle rear end (15), the rotatable retract control panel (18) is detachably connected to the handle rear end (15) by a buckle (31).

6. The handle for a medical implant delivery system of claim 5, wherein a groove is provided on the buckle (31), the retract control panel (18) snaps into the groove on the buckle (31), the retract control panel (18) is ring-shaped, a second notch (29) and a third notch (30) for the distal end of the buckle (31) to pass through are provided inside the retract control panel (18), the shape of the second notch (29) matches the proximal end of the laminated pull ring (12) or the rear release pull ring (14), when the laminated pull ring (12) or the rear release pull ring (14) is inserted into the second notch (29), the position of the third notch (30) and the buckle (31) are staggered.

7. The handle for a medical implant delivery system of claim 6, wherein when the retract control panel (18) rotates to the third notch (30) and overlaps with the buckle (31), the retract control panel (18) is separated from the buckle (31) making the inner tube end connector (13) is in a retractable state.

8. The handle for a medical implant delivery system of claim 1, wherein the surface of the outer tube (6) is coated with a hydrophilic coating.

## Patentansprüche

1. Griff für ein System zur Abgabe eines medizinischen Implantats, umfassend einen vorderen Griff (8) und einen hinteren Griff (16), wobei der vordere Griff (8) und der hintere Griff (16) intern mit einer Führungsstange (1) versehen ist, wobei ein Gleitblock (2) auf der Führungsstange (1) vorgesehen ist, wobei der vordere Griff (8) und ein hinteres Griffende (15) als Ganzes mithilfe der Führungsstange (1) fest verbunden sind, wobei der Gleitblock (2) und eine Außenröhre (6) als Ganzes mithilfe einer Außenröhrenverbindungsteils fest verbunden sind, wobei der Gleitblock (2) und der hintere Griff (16) drehbar verbunden sind, wobei sich der hintere Griff (16) dreht, um zu ermöglichen, dass der Gleitblock (2) die Außenröhre (6) antreibt, um zu einem distalen Ende auf der Führungsstange (1) hin zu gleiten, wobei ein Rückdrehsperrschalter (17) und eine elastische Rückdrehsperrplatte (3), die eine Drehrichtung des hinteren Griffs (16) einschränkt, an dem Verbindungspunkt des vorderen Griffs (8) und des hinteren Griffs (16) vorgesehen sind,
wobei ein erster Ansatz (23) auf dem Rückdrehsperrschalter (17) vorgesehen ist, wobei eine erste Nut (24), die mit dem ersten Ansatz (23) zusammenpasst, auf dem hinteren Griff (16) vorgesehen ist, wobei, wenn der Rückdrehsperrschalter (17) geschlossen ist, der erste Ansatz (23) der Rückdrehsperrschalters (17) in die erste Nut (24) des hinteren Griffs (16) eingesetzt wird und der hintere Griff (16) dann nicht gedreht werden kann, wobei, nachdem der Rückdrehsperrschalter (17) eingeschaltet wurde, der erste Ansatz (23) des Rückdrehsperrschalters (17) und die erste Nut (24) des hinteren Griffs (16) getrennt werden und der hintere Griff (16) dann gedreht werden kann,
wobei die elastische Rückdrehsperrplatte (3) einen geneigten Arm mit Elastizität aufweist, wobei ein Vorsprung an dem Ende des geneigten Arms vorliegt, der auf einer Seite geneigt ist und auf der anderen Seite gerade ist, wobei der Vorsprung in die erste Nut (24) an dem proximalen Ende des hinteren Griffs (16) eingesetzt wird, wobei eine Vielzahl der ersten Nuten (24) vorliegt, die in einem Kreis um einen Umfang des proximalen Endes des hinteren Griffs (16) eingerichtet ist.

2. Griff für ein System zur Abgabe eines medizinischen Implantats nach Anspruch 1, wobei ein Bedienteil (11) drehbar auf der Außenumfangsfläche des hinteren Griffendes (15) vorgesehen ist, wobei ein laminierter Zugring (12) und ein hinterer Freigabezugring (14) sequenziell auf dem hinteren Griffende (15) entlang der Drehrichtung des Bedienteils (11) vorgesehen ist, wobei ein innerer Röhrenendverbinder (13) intern in dem hinteren Griffende (15) vorgesehen ist, wobei der laminierte Zugring (12) und der hintere Freigabezugring (14) abnehmbar mit dem Bedienteil (11) verbunden sind.

3. Griff für ein System zur Abgabe eines medizinischen Implantats nach Anspruch 2, wobei das Bedienteil (11) eine hohle zylindrische Form aufweist, wobei ein zweiter Ansatz (25) im Inneren des Bedienteils (11) vorgesehen ist wobei eine zweite Nut (32), die mit dem zweiten Ansatz (25) zusammenpasst, auf dem laminierten Zugring (12) und dem hinteren Freigabezugring (14) vorgesehen ist, wobei eine einzigartige Kerbe, durch die der laminierte Zugring (12) des proximalen Endes und der hintere Freigabezugring (14) hindurchtreten können, auf dem zweiten Ansatz (25) im Inneren der Bedienplatte (11) ausgebildet ist, wobei ein invertierter Einwegezahn (33) im Inneren der Bedienplatte (11) vorgesehen ist.

4. Griff für ein System zur Abgabe eines medizinischen Implantats nach Anspruch 3, wobei eine Prallplatte (22), die nach innen vorragt, an dem proximalen oder dem distalen Ende an der Kerbe des Bedienteils (11) vorgesehen ist, wobei, wenn sich die Prallplatte (22) zu dem laminierten Zugring (12) oder dem hinteren Freigabezugring (14) dreht, die Prallplatte (22) gegen den laminierten Zugring (12) oder den hinteren Freigabezugring (14) ist, was bewirkt, dass das Bedienteil (11) aufhört, sich zu drehen, wobei die Kerbe des zweiten Ansatzes (25) des Bedienteils (11) auf den laminierten Zugring (12) oder den hinteren Freigabezugring (14) ausgerichtet ist.

5. Griff für ein System zur Abgabe eines medizinischen Implantats nach Anspruch 4, wobei ein drehbares Rückzugsbedienteil (18) zwischen dem Bedienteil (11) und dem hinteren Griffende (15) vorgesehen ist, wobei das drehbare Rückzugsbedienteil (18) durch eine Schnalle (31) abnehmbar mit dem hinteren Griffende (15) verbunden ist.

6. Griff für ein System zur Abgabe eines medizinischen Implantats nach Anspruch 5, wobei eine Nut auf der Schnalle (31) vorgesehen ist, wobei das Rückzugsbedienteil (18) in die Nut auf der Schnalle (31) einrastet, wobei das Rückzugsbedienteil (18) ringförmig ist, wobei eine zweite Kerbe (29) und eine dritte Kerbe (30), damit das distale Ende der Schnalle (31) hindurchtritt, im Inneren des Rückzugsbedienteils (18) vorgesehen sind, wobei die Form der zweiten Kerbe (29) dem proximalen Ende des laminierten Zugrings (12) oder des hinteren Freigabezugrings (14) entspricht, wobei, wenn der laminierte Zugring (12) oder der hintere Freigabezugring (14) in die zweite Kerbe (29) eingesetzt wird, die Position der dritten Kerbe (30) und die Schnalle (31) gestaffelt sind.

7. Griff für ein System zur Abgabe eines medizinischen Implantats nach Anspruch 6, wobei, wenn sich das Rückzugsbedienteil (18) zu der dritten Kerbe (30) dreht und mit der Schnalle (31) überlappt, das Rückzugsbedienteil (18) von der Schnalle (31) getrennt wird, was bewirkt, dass der innere Röhrenendverbinder (13) in einem zurückziehbaren Zustand ist.

8. Griff für ein System zur Abgabe eines medizinischen Implantats nach Anspruch 1, wobei die Fläche der Außenröhre (6) mit einer hydrophilen Beschichtung beschichtet ist.

## Revendications

1. Poignée pour un système de pose d'implant médical, comprenant une poignée avant (8) et une poignée arrière (16), la poignée avant (8) et la poignée arrière (16) sont pourvues à l'intérieur d'une tige de guidage (1), un bloc coulissant (2) est prévu sur la tige de guidage (1), la poignée avant (8) et une extrémité arrière de poignée (15) sont reliées à demeure dans leur ensemble au moyen de la tige de guidage (1), le bloc coulissant (2) et un tube externe (6) sont reliés à demeure dans leur ensemble au moyen d'une partie de raccordement de tube externe, le bloc coulissant (2) et la poignée arrière (16) sont reliés en rotation, la poignée arrière (16) tourne pour permettre au bloc coulissant (2) d'entraîner le tube externe (6) à coulisser vers l'extrémité distale sur la tige de guidage (1), dans laquelle un commutateur anti-inversion de rotation (17) et une plaque élastique anti-inversion de rotation (3) qui limite un sens de rotation de la poignée arrière (16) sont prévus au niveau du point de raccordement de la poignée avant (8) et de la poignée arrière (16),
un premier bossage (23) est prévu sur le commutateur anti-inversion de rotation (17), une première rainure (24) correspondant au premier bossage (23) est prévue sur la poignée arrière (16), lorsque le commutateur anti-inversion de rotation (17) est fermé, le premier bossage (23) du commutateur anti-inversion de rotation (17) est inséré dans la première rainure (24) de la poignée arrière (16), et ensuite la poignée arrière (16) ne peut pas être tournée, après l'activation du commutateur anti-inversion de rotation (17), le premier bossage (23) du commutateur anti-inversion de rotation (17) et la première rainure (24) de la poignée arrière (16) sont séparés, puis la poignée arrière (16) peut être tournée,
la plaque élastique anti-inversion de rotation (3) présente un bras incliné à élasticité, il y a une saillie à l'extrémité du bras incliné qui est inclinée d'un côté et droite de l'autre côté, la saillie est insérée dans la première rainure (24) à l'extrémité proximale de la poignée arrière (16), dans laquelle il y a une pluralité de premières rainures (24) agencées en cercle autour d'une circonférence de l'extrémité proximale de la poignée arrière (16).

2. Poignée pour un système de pose d'implant médical selon la revendication 1, dans laquelle un panneau de commande (11) est prévu en rotation sur la surface circonférentielle extérieure de l'extrémité arrière de poignée (15), un anneau de traction laminé (12) et un anneau de traction de libération arrière (14) sont prévus séquentiellement sur l'extrémité arrière de poignée (15) le long du sens de rotation du panneau de commande (11), un connecteur d'extrémité de tube interne (13) est prévu à l'intérieur dans l'extrémité arrière de poignée (15), l'anneau de traction laminé (12) et l'anneau de traction de libération arrière (14) sont reliés de manière détachable au panneau de commande (11).

3. Poignée pour un système de pose d'implant médical selon la revendication 2, dans laquelle le panneau de commande (11) présente une forme cylindrique creuse, un second bossage (25) est prévu à l'intérieur du panneau de commande (11), une seconde rainure (32) correspondant au second bossage (25) est prévue sur l'anneau de traction laminé (12) et l'anneau de traction de libération arrière (14), une encoche unique à travers laquelle l'anneau de traction laminé (12) d'extrémité proximale et l'anneau de traction de libération arrière (14) peuvent passer est formée sur le second bossage (25) à l'intérieur de la plaque de commande (11), une dent inversée unidirectionnelle (33) est prévue à l'intérieur de la plaque de commande (11).

4. Poignée pour un système de pose d'implant médical selon la revendication 3, dans laquelle une plaque de déflecteur (22) faisant saillie vers l'intérieur est prévue à l'extrémité proximale ou distale au niveau de l'encoche du panneau de commande (11), lorsque la plaque de déflecteur (22) tourne vers l'anneau de traction laminé (12) ou l'anneau de traction de libération arrière (14), la plaque de déflecteur (22) se situe contre l'anneau de traction laminé (12) ou l'anneau de traction de libération arrière (14) provoquant l'arrêt de la rotation du panneau de commande (11), l'encoche du second bossage (25) du panneau de commande (11) est alignée avec l'anneau de traction laminé (12) ou avec l'anneau de traction de libération arrière (14).

5. Poignée pour un système de pose d'implant médical selon la revendication 4, dans laquelle un panneau de commande de rétraction (18) rotatif est prévu entre le panneau de commande (11) et l'extrémité arrière de poignée (15), le panneau de commande de rétraction rotatif (18) est relié de manière détachable à l'extrémité arrière de poignée (15) par une boucle (31).

6. Poignée pour un système de pose d'implant médical selon la revendication 5, dans laquelle une rainure est prévue sur la boucle (31), le panneau de commande de rétraction (18) s'emboîte dans la rainure sur la boucle (31), le panneau de commande de rétraction (18) est en forme d'anneau, une deuxième encoche (29) et une troisième encoche (30) pour que l'extrémité distale de la boucle (31) passe à travers sont prévues à l'intérieur du panneau de commande de rétraction (18), la forme de la deuxième encoche (29) correspond à l'extrémité proximale de l'anneau de traction laminé (12) ou de l'anneau de traction de libération arrière (14), lorsque l'anneau de traction laminé (12) ou l'anneau de traction de libération arrière (14) est inséré dans la deuxième encoche (29), la position de la troisième encoche (30) et de la boucle (31) sont décalées.

7. Poignée pour un système de pose d'implant médical selon la revendication 6, dans laquelle lorsque le panneau de commande de rétraction (18) tourne jusqu'à la troisième encoche (30) et chevauche la boucle (31), le panneau de commande de rétraction (18) est séparé de la boucle (31) rendant le connecteur d'extrémité de tube interne (13) dans un état rétractable.

8. Poignée pour un système de pose d'implant médical selon la revendication 1, dans laquelle la surface du tube externe (6) est revêtue d'un revêtement hydrophile.
